**Europäisches Patentamt**

**European Patent Office**

**Office européen des brevets**

(11) Veröffentlichungsnummer: **0 429 832 B1**

## (12) EUROPÄISCHE PATENTSCHRIFT

(45) Veröffentlichungstag der Patentschrift: **22.06.94**

(51) Int. Cl.⁵: **A61K 9/20**

(21) Anmeldenummer: **90119915.8**

(22) Anmeldetag: **17.10.90**

---

(54) **Nicht wirkstoffretardierender Pressling, Verfahren zu dessen Herstellung und Verwendung von Polyhydroxybuttersäure zur Herstellung eines solchen Presslings.**

---

(30) Priorität: **30.11.89 AT 2734/89**

(43) Veröffentlichungstag der Anmeldung:
**05.06.91 Patentblatt 91/23**

(45) Bekanntmachung des Hinweises auf die
Patenterteilung:
**22.06.94 Patentblatt 94/25**

(84) Benannte Vertragsstaaten:
**CH DE ES GB GR LI**

(56) Entgegenhaltungen:
**EP-A- 0 262 583**

**Voigt, Lehrbuch der Pharmazeutischen Technologie, 4 Auflage, 1982**

(73) Patentinhaber: **PCD Polymere Gesellschaft m.b.H.**
**Danubiastrasse 21-25**
**A-2323 Schwechat-Mannswörth(AT)**

(72) Erfinder: **Korsatko-Wabnegg, Brigitta, Mag. Dr.**
**Kärntnerstrasse 432**
**A-8054 Graz(AT)**
Erfinder: **Korsatko, Werner, Mag. Dr.**
**Kärntnerstrasse 432**
**A-8054 Graz(AT)**

(74) Vertreter: **Kunz, Ekkehard, Dr.**
**Chemie Linz Ges.m.b.H.**
**Abteilung Patente**
**St. Peter-Str. 25**
**A-4021 Linz (AT)**

EP 0 429 832 B1

**Beschreibung**

Die Erfindung betrifft einen Preßling für die orale Applikation, der keine wirkstoffretardierenden Eigenschaften besitzt, bestehend aus einem Homo- und/oder Copolymer der D(-)-3-Hydroxybuttersäure (Poly-HB), mindestens einem Zerfallsmittel, mindestens einem pharmazeutischen Wirkstoff und gegebenenfalls in der Galenik üblichen Hilfsstoffen, Verfahren zu dessen Herstellung und die Verwendung von Poly-HB zur Herstellung eines solchen Preßlings.

In EP-A-0 108 882 ist beschrieben, daß Poly-D(-)-3-hydroxybuttersäure geringe elastische Eigenschaften, geringe elektrostatische Aufladungstendenz und gute Schmier- und Gleiteigenschaften besitzt, und daß aus Poly-D(-)-3-hydroxybuttersäure und einem pharmazeutischen Wirkstoff Preßlinge mit guten wirkstoffretardierenden Eigenschaften hergestellt werden können.

Solche Retardformen werden verwendet, wenn der pharmazeutische Wirkstoff über einen längeren Zeitraum hinweg verzögert abgegeben werden soll. In sehr vielen Fällen ist es jedoch notwendig, daß der Wirkstoff nicht retardiert, sondern sofort freigesetzt wird. Üblicherweise wird zur Herstellung solcher nicht retardierender Preßlinge der pharmazeutische Wirkstoff vor dem Verpressen mit Hilfsstoffen vermischt, wobei jedoch die Eignung dieser Hilfsstoffe, etwa bezüglich ihrer Komprimierungseigenschaften nicht immer völlig zufriedenstellend ist.

Es wurde nun unerwarteterweise gefunden, daß man Poly-HB trotz ihrer retardierenen Eigenschaften zur Herstellung von verpreßten Arzneiformen, aus denen im Anwendungsfall der Wirkstoff sofort freigesetzt und nicht retardiert wird, verwenden kann, wenn man eine Mischung aus pharmazeutischem Wirkstoff, Poly-HB und einem Zerfallsmittel gemeinsam verpreßt. Überraschenderweise wird dennoch eine stabile Matrix gebildet, in die der pharmazeutische Wirkstoff eingebettet ist, wobei der Zusatz des Zerfallsmittels die guten Verpressungseigenschaften der Mischung nicht beeinträchtigt. Solche verpreßte Arzneimittel stellen somit eine Bereicherung der Technik dar.

Gegenstand der Erfindung ist daher ein Preßling für die orale Applikation mit nicht retardierter Wirkstofffreisetzung, der dadurch gekennzeichnet ist, daß er ein Homo- und/oder Copolymer der D(-)-3-Hydroxybuttersäure, mindestens ein festes Zerfallsmittel und mindestens einen festen, pharmazeutischen Wirkstoff enthält.

Unter Homo- und/oder Copolymer des D(-)-3-Hydroxybuttersäure (Poly-HB) sind sowohl Homo-, als auch Copolymere der D(-)-3-Hydroxybuttersäure und Mischungen daraus zu verstehen, wobei Homopolymere bevorzugt sind. Homopolymere der D(-)-3-Hydroxybuttersäure können beispielsweise nach der in EP-A-0 144 017, Copolymere nach der in EP-A-0 052 459 beschriebenen Verfahrensweise hergestellt werden. Die üblicherweise verwendete Poly-HB besitzt ein Molekulargewicht von etwa 25 000 bis 1 000 000, bevorzugt von etwa 50 000 bis 800 000. Poly-HB liegt im erfindungsgemäßen Preßling in Pulverform oder vorgranuliert zu einem Anteil von etwa 10 bis 90 Gewichtsprozent vor.

Unter Zerfallsmittel sind Zusätze zu verstehen, die die Retardeigenschaften der Poly-HB, nicht aber deren gute Komprimierungseigenschaften aufheben. Beispiele für solche Zerfallsmittel, die oft auch als Sprengmittel bezeichnet werden, sind etwa im Lehrbuch der pharmazeutischen Technologie, R. Voigt, 5. Auflage, Verlag Chemie, Weinheim (1984), Seiten 182 ff, geoffenbart. Bevorzugt liegen im erfindungsgemäßen Preßling Zerfallsmittel vor, die die Kapillarität des Preßlings erhöhen, Feuchtigkeit absorbieren und quellen, wie beispielsweise mikrokristalline Cellulose, quervernetzte Natriumcarboxymethylcellulose, quervernetztes Polyvinylpyrrolidon, Natriumcarboxymethylstärke, Calziumsalz des Polycarboxymethylethers der Cellulose.

Die Menge des Zerfallsmittels im erfindungsgemäßen Preßling ist von der Menge der verwendeten Poly-HB, sowie von der Sprengkraft des verwendeten Zerfallsmittels abhängig. Die benötigte Menge ist durch einfache Versuche für jedes Zerfallsmittel zu ermitteln und liegt im allgemeinen im Preßling zwischen 0,5 bis zu 70 Gew.%.

Unter festen pharmazeutischen Wirkstoffen sind feste pharmazeutische Wirkstoffe oder Mischungen derselben zu verstehen, wobei die Wirkstoffe im Preßling in freier Form, in Form pharmakologisch verträglicher Salze, in Pulverform oder granuliert vorliegen können.
Der Anteil des Wirkstoffes im erfindungsgemäßen Preßling hängt von der Art des Wirkstoffes und seinem Einsatzgebiet ab. Im allgemeinen enthält der Preßling 5 bis 80 Gew.% Wirkstoff.

Der Preßling kann ausschließlich aus Poly-HB, Wirkstoff und Zerfallsmittel bestehen, es ist aber auch möglich, daß er darüber hinaus übliche galenische Hilfsstoffe, wie z. B. Füllmittel, Bindemittel, Gleit- und Schmiermittel oder Farbstoffe enthält, die in Pulverform oder vorgranuliert vorliegen können. Es hat sich herausgestellt, daß es möglich ist, einen Teil der Poly-HB durch Lactose zu ersetzen, welche billiger ist als Poly-HB. Ein Mindestanteil von etwa 10 Gew.% Poly-HB im Preßling muß jedoch zur Ausbildung einer stabilen Matrix vorhanden sein. Beispiele für Hilfsstoffe, die im erfindungsgemäßen Preßling vorliegen können, sind im Lehrbuch der pharmazeutischen Technologie, R. Voigt, 5. Auflage, Verlag Chemie Weinheim, (1984), Seite 178 ff, insbesondere unter Punkt 8.5.2., 8.5.3. und 8.5.4. beschrieben.

Die Zerfallseigenschaften des Preßlings hängen von der Art und Menge der verwendeten Bestandteile des Preßlings, von deren Korngröße und vom verwendeten Preßdruck ab. Im allgemeinen zerfällt der Preßling umso schneller, je höher der Anteil an Zerfallsmittel im Preßling und je geringer der Preßdruck ist, wobei der Preßdruck üblicherweise nicht weniger als 0,5 Tonnen/Tablette betragen soll, da ansonsten die Bruchfestigkeit des Preßlings zu gering ist. Die gewünschte hohe Zerfallsgeschwindigkeit kann also durch Variation der verschiedenen Parameter durch einfache Versuche ermittelt werden.

Ein weiterer Gegenstand der Erfindung ist ein Verfahren zur Herstellung eines Preßlings mit nicht retardierter Wirkstofffreisetzung, das dadurch gekennzeichnet ist, daß ein Homo- und/oder Copolymer der D(-)-3-Hydroxybuttersäure mit mindestens einem festen Zerfallsmittel, mindestens einem festen, pharmazeutischen Wirkstoff mit oder ohne in der Galenik übliche Hilfsstoffe durchmischt und die Mischung mit oder ohne Vorgranulierung verpreßt wird.

Das Durchmischen kann bei geringen Mengen mittels Pulverreibschale und Pistill oder einer Pulvermischdose erfolgen. Große Ansätze lassen sich mit Hilfe von rotierenden Trommeln, Schaufelmischern, Tellermischern, Mischschnecken, Ribbonmischern, Konusmischern, Doppelkonusmischern und V-Mischern (Twin-Shellblender) gut durchmischen. Die erhaltenen Mischungen werden zu Tabletten oder Drageekernen beliebiger Form und Größe verpreßt. Es ist auch möglich, kleine Komprimate zur Abfüllung in Kapseln oder zur Herstellung von Mehrschichttabletten, herzustellen. Bevorzugt werden die Mischungen direkt und ohne weiter Behandlung verpreßt. Sie können aber auch vor dem Verpressen auf übliche Weise vorgranuliert werden. Die Herstellung der Preßlinge ist mit allen herkömmlichen Tablettenpressen, wie beispielsweise Exzenterpressen, Rundläuferpressen, hydraulischen Pressen möglich, wobei der Preßdruck etwa über einen Bereich von 0,5 bis 10 Tonnen, das sind 49,05 - 981 N pro Tablette variiert werden kann.

Der erfindungsgemäße Preßling kann infolge der matrixbildenden Eigenschaften der Poly-HB durch einfaches Verpressen der Pulvermischungen hergestellt werden und besitzt eine ausgezeichnete Lagerstabilität. Es ist nicht notwendig, daß die Pulvermischung vor dem Verpressen vorgranuliert wird.

In den Beispielen wurde eine Poly-HB eines Molekulargewichts von 97.000 und einer Korngrößenfraktion von 125 bis 200 Mikrometer verwendet. Als Wirkstoff wurde 2-Amino-N-(2-(2,5-dimethoxyphenyl)-2-hydroxyethyl)acetamid.HCl (Midodrin.HCl) ausgewählt.

Die jeweiligen Bestandteile wurden homogen vermischt, und ohne Vorgranulierung mittels einer elektrohydraulischen Presse zu Tabletten verpreßt. Das Durchschnittsgewicht der Tabletten betrug, wenn nicht anders angegeben, 100 ± 3 mg, der Durchmesser der Tabletten 7 mm, die Höhe etwa 2 mm.

Die Freisetzungsrate des Wirkstoffes wurde teilweise nach der half-change Methode, bei der der pH-Wert kontinuierlich innerhalb von 8 Stunden von pH 1,3 auf pH 7,3 erhöht wird, wobei in Abständen von 30 Minuten auf die freigesetzte Wirkstoffmenge untersucht wurde, bestimmt. Die quantitative Erfassung von Midodrin erfolgte spektralphotometrisch bei 289 nm.

Die Messung der Zerfallszeiten wurde auch nach der im Europäischen Arzneibuch, Band III, (1981), Seite 78ff geoffenbarten Methode bei verschiedenen pH-Werten vorgenommen. Dabei wurden die jeweiligen Rückstände nach der jeweiligen Zeit getrocknet und gravimetrisch bestimmt. Bei Tabletten, die Midodrin.HCl enthielten, erfolgte die quantitative Erfassung der freigesetzten Wirkstoffmenge spektralphotometrisch bei 289 nm (Zerfallstest).

| | |
|---|---|
| Puffer pH 1: | Puffer Titrisol Merck Art. 9881 |
| Puffer pH 4: | Puffer Titrisol Merck Art. 9884 |
| Puffer pH 7: | Puffer Titrisol Merck Art. 9887 |
| Puffer pH 7,4: | Phosphatpuffer nach Sörensen |
| $Na_2HPO_4.12H_2O$ | 41,246 g |
| $KH_2PO_4$ | 2,8 g |
| aqua bidest | ad 1000,0 ml |

Beispiel 1

Herstellung von Placebotabletten aus Poly-HB und Primojel (Natriumcarboxymethylstärke, Fa. W.A. Scholten's Chem. Fabriken N.V., Foxhol, NL)

Aus Poly-HB und Primojel wurden Tabletten verschiedener Zusammensetzung bei einem Preßdruck von 2 Tonnen pro Tablette hergestellt. Die Tabletten besaßen ein Gewicht von 150 mg, einen Durchmesser von 8 mm und eine Höhe von 2,5 ± 1 mm. Die Schüttdichte stieg von 0,30 g/ml (10 Gew.% Primojel) auf 0,39 g/ml (50 Gew.% Primojel), die Rütteldichte von 0,37 g/ml (10 Gew.% Primojel) auf 0,56 g/ml (50 Gew.% Primojel), die Bruchfestigkeit betrug mehr als 20 kp, bei 50 % Primojelanteil knapp 20 kp. Die Zerfallszeiten wurden mit Hilfe des Zerfallstests bei verschiedenen pH-Werten bestimmt. Die Ergebnisse wurden in Tabelle 1 zusammengefaßt:

## Tabelle 1

| Primojel Gew.% | pH 1 | pH 4 | pH 7,4 |
|---|---|---|---|
| | Zerfallszeit in Minuten | | |
| 10 | 241 | 260 | 260 |
| 20 | 62 | 70 | 67 |
| 30 | 30 | 30 | 29 |
| 40 | 18 | 20 | 17 |
| 50 | 10 | 11 | 13 |

Beispiel 2

Herstellung von Tabletten, bestehend aus Poly-HB, Primojel und 2-Amino-N-(2-(2,5-dimethoxyphenyl)-2-hydroxyethyl)acetamid.HCl (Midodrin.HCl)

Aus 131,63 Gewichtsteilen (Gew.T) Poly-HB, 3,37 Gew.T Primojel und 15 Gew.T Midodrin.HCl wurden bei einem Preßdruck von 2 Tonnen pro Tablette Tabletten eines Gewichtes von etwa 150 mg, eines Durchmessers von 8mm, einer Höhe von 2,5 mm, einer Schüttdichte von 0,27 g/ml, einer Rütteldichte von 0,40 g/ml und einer Bruchfestigkeit von mehr als 20 kp hergestellt, die 2,25 Gew.% Primojel enthielten. Die Freisetzung des Wirkstoffes wurde nach der half-change Methode geprüft. Danach waren nach 30 Minuten etwa 20 %, nach 1 1/2 Stunden etwa 35 % und nach 4 Stunden praktisch 100 % des Wirkstoffes freigesetzt.

Beispiel 3

Herstellung von Tabletten, bestehend aus Poly-HB, Primojel und Midodrin.HCl

Aus 78,56 Gew.T Poly-HB, 6,44 Gew.T Primojel und 15 Gew.T Midodrin.HCl wurden bei einem Preßdruck von 2 Tonnen pro Tablette Tabletten mit einer Bruchfestigkeit von 16,2 kp hergestellt, die 6,4 Gew.% Primojel enthielten. Die Freisetzung des Wirkstoffes wurde nach der half-change Methode geprüft. Danach waren nach 30 Minuten über 40 %, nach 1 1/2 Stunden fast 90 % und nach 2 Stunden praktisch 100 % des Wirkstoffes freigesetzt.

Beispiel 4

Herstellung von Tabletten, bestehend aus Poly-HB, Primojel und Midodrin.HCl

Aus 128,25 Gew.T Poly-HB, 15 Gew.T Midodrin.HCl und 6,75 Gew.T Primojel wurden Tabletten eines Gewichtes von 150 mg, eines Durchmessers von 8mm und einer Rütteldichte von 0,40 g/ml bei verschiedenen Preßdrucken hergestellt, die 4,5 Gew.% Primojel enthielten. Die Bruchfestigkeit war von den verwendeten Preßdrucken praktisch unabhängig und betrug zwischen 16 und 20 kp. Mit Hilfe des Zerfallstestes wurden bei einem pH-Wert von 1 die Zerfallszeiten ermittelt, welche in Tabelle 2 angeführt sind.

## Tabelle 2

Preßdruck (Tonnen/Tablette)

| 0,5 | 1 | 2 | 3 | 4 | 5 |
|---|---|---|---|---|---|
| Freigesetzter Wirkstoff (Gew.%): | | nach 30 Minuten | | | |
| 86,8 | 46,2 | 38,5 | 35,8 | 29,1 | 28,1 |
| | | | : | nach 90 Minuten | |
| 95,5 | 88 | 84 | 88 | 76,5 | 65,8 |
| | | | : | nach 120 Minuten | |
| 97 | 95 | 95 | 95 | 88,5 | 79,2 |

Beispiel 5

Herstellung von Tabletten, bestehend aus Poly-HB, Polyplasdone XL (quervernetztes Polyvinylpyrrolidon, Fa. General Aniline and Film Corporation, N.Y.), und Midodrin.HCl

83,64 Gew.T Poly-HB, 1,36 Gew.T Polyplasdone XL und 15 Gew.T Midodrin.HCl wurden bei einem Preßdruck von 2 Tonnen pro Tablette zu Tabletten verpreßt, die eine Bruchfestigkeit von 18 kp aufwiesen. Die Freisetzung des Wirkstoffes wurde nach der half-change Methode geprüft. Nach einer halben Stunde waren 46 %, nach 1 1/2 Stunden 90 % und nach 2 Stunden fast 100 % des Wirkstoffes freigesetzt

Beispiel 6

Herstellung von Tabletten, bestehend aus Poly-HB, Ac-Di-Sol (Natriumcarboxymethylcellulose, Fa. Lehmann und Voss, Hamburg, BRD) und Midodrin.HCl

83,64 Gew.T Poly-HB, 1,36 Gew.T Ac-Di-Sol und 15 Gew.T Midodrin.HCl wurden bei einem Preßdruck von 2 Tonnen pro Tablette zu Tabletten verpreßt, die eine Bruchfestigkeit von 18,3 kp und 1,36 Gew.% Ac-Di-Sol aufwiesen. Die Freisetzung des Wirkstoffes wurde nach der half-change Methode geprüft. Nach einer halben Stunde waren 45,6 % nach 1 1/2 Stunden mehr als 90 % und nach 2 Stunden praktisch 100 % des Wirkstoffes freigesetzt.

Beispiel 7

Herstellung von Placebo-Tabletten, bestehend aus Poly-HB und Ac-Di-Sol

Aus verschiedenen Gewichtsteilen Poly-HB und Ac-Di-Sol wurden Tabletten einer steigenden Schüttdichte von 0,26 g/ml (10 Gew.% Ac-Di-Sol) bis 0,37 g/ml (50 Gew.% Ac-Di-Sol) einer steigenden Rütteldichte von 0,40 g/ml (10 Gew.% Ac-Di-Sol) bis 0,43 g/ml (50 Gew.% Ac-Di-Sol) und einer Bruchfestigkeit von mehr als 20 kp hergestellt. Die Zerfallszeiten wurden bei verschiedenen pH-Werten mittels des Zerfallstestes ermittelt; sie sind in Tabelle 3 zusammengefaßt.

## Tabelle 3

| Ac-Di-Sol (Gew.%) | pH-Wert | Zerfallszeit (Minuten) |
|---|---|---|
| 10 | 1 | 90 |
|  | 4 | 63 |
|  | 7 | 59 |
| 20 | 1 | 16 |
|  | 4 | 12 |
|  | 7 | 11 |
| 30 | 1 | 10 |
|  | 4 | 8 |
|  | 7 | 7 |
| 40 | 1 | 7 |
|  | 4 | 6 |
|  | 7 | 6 |
| 50 | 1 | 3,5 |
|  | 4 | 3 |
|  | 7 | 3 |

Beispiel 8

Herstellung von Placebo-Tabletten aus Poly-HB und E.C.G. 505 (Calziumsalz des Polycarboxymethylethers der Cellulose, Fa. Lehmann und Voss, Hamburg, BRD)

Aus verschiedenen Gewichtsanteilen Poly-HB und E.C.G. 505 wurden bei einem Preßdruck von 2 Tonnen pro Tablette Tabletten eines Gewichtes von 150 mg und eines Durchmessers von 8 mm hergestellt, die eine steigende Schüttdichte von 0,28 g/ml (10 Gew.% E.C.G. 505) bis 0,33 g/ml (50 Gew.% E.C.G. 505), eine steigende Rütteldichte von 0,34 g/ml (10 Gew.% E.C.G.505) bis 0,49 g/ml (50 Gew.% E.C.G. 505) und eine Bruchfestigkeit, die in allen Fällen höher war als 20 kp, aufwiesen. Mit Hilfe des Zerfallstestes wurden die Zerfallszeiten bei verschiedenen pH-Werten ermittelt, die in Tabelle 4 aufgeführt sind.

## Tabelle 4

| E.C.G.505 (Gew.%) | pH 1 | pH 4 | pH 7,4 |
|---|---|---|---|
| | Z e r f a l l s z e i t (min) | | |
| 10 | 260 | 226 | 211 |
| 20 | 139 | 113 | 111 |
| 30 | 45 | 43 | 52 |
| 40 | 35 | 31 | 30 |
| 50 | 30 | 25 | 22 |

Beispiel 9

Herstellung von Tabletten, bestehend aus Poly-HB, E.C.G. 505 und Midodrin.HCl

Aus 128,25 Gew.T Poly-HB, 6,75 Gew.T E.C.G. 505 und 15 Gew.T Midodrin.HCl wurden bei einem Preßdruck von 2 Tonnen pro Tablette Tabletten eines Gewichtes von 150 mg hergestellt, die eine Schüttdichte von 0,24 g/ml, eine Rütteldichte von 0,35 g/ml, und eine Bruchfestigkeit von mehr als 20 kp aufwiesen. Die Freisetzungsrate des Midodrin.HCl wurde mit der half-change Methode bestimmt. Dabei waren nach einer halben Stunde 26 % nach 1 1/2 Stunden 60 % und nach 3 Stunden praktisch 100 % des Wirkstoffes freigesetzt.

Beispiel 10

Herstellung von Tabletten, bestehend aus Poly-HB, E.C.G. 505 und Midodrin.HCl

Aus 128,25 Gew.T Poly-HB, 6,75 Gew.T E.C.G. 505 und 15 Gew.T Midodrin.HCl wurden Tabletten mit den in Beispiel 9 angeführten Eigenschaften bei verschiedenen Preßdrucken hergestellt. Die Freisetzung des Wirkstoffes wurde bei einem pH-Wert von 1 mittels des Zerfalltestes geprüft. Die Ergebnisse sind in Tabelle 5 zusammengefaßt.

## Tabelle 5

| Preßdruck (Tonne/Tablette) | | | | | |
|---|---|---|---|---|---|
| 0,5 | 1 | 2 | 3 | 4 | 5 |
| Freigesetzter Wirkstoff (Gew.%): nach 30 Minuten | | | | | |
| 80,8 | 41,8 | 33,1 | 34,2 | 28,7 | 26,5 |
| : nach 90 Minuten | | | | | |
| 98,4 | 83,5 | 81,3 | 87,6 | 77,9 | 77,7 |
| : nach 120 Minuten | | | | | |
| 98,9 | 93,4 | 93,6 | 95,8 | 91,4 | 86,3 |

EP 0 429 832 B1

Beispiel 11

Herstellung von Placebo-Tabletten aus Poly-HB und Avicel pH (mikrokristalline Cellulose, Fa. Lehmann und Voss, Hamburg, BRD)

7 Gew.T Poly-HB und 3 Gew.T Avicel einer Korngröße von 71 bis 100 $\mu$m wurden bei einem Preßdruck von 2 Tonnen pro Tablette zu Tabletten, die 30 Gew.% Avicel enthielten, verpreßt. Die Schüttdichte betrug 0,27 g/ml, die Rütteldichte 0,33 g/ml und die Bruchfestigkeit mehr als 20 kp. Die Tabletten begannen im Zerfallstest bei einem pH-Wert von 7 nach 15 Minuten, bei einem pH-Wert von 1 nach 9 Minuten zu zerfallen.

Tabletten, bestehend aus Poly-HB und Avicel PH einer Korngröße von 71 bis 100 $\mu$m im Gewichtsverhältnis 9 : 1, die auf die oben beschriebene Art und Weise hergestellt wurden und die eine Schüttdichte von 0,27 g/ml, eine Rütteldichte von 0,34 g/ml und eine Bruchfestigkeit von mehr als 20 kp aufwiesen, zerfielen dagegen bei der in vitro Prüfung innerhalb von 24 Stunden weder bei einem pH-Wert von 7 noch bei einem pH-Wert von 1.

Beispiel 12

Herstellung von Placebo-Tabletten, bestehend aus Poly-HB und Avicel PH

Tabletten bestehend aus Poly-HB und Avicel im Gewichtsverhältnis 6 : 4 und 5 :5 wurden bei einem Preßdruck von 2 Tonnen pro Tablette hergestellt. Deren Schüttdichte stieg von 0,27 g/ml (40 Gew.% Avicel) auf 0,31 g/ml (50 Gew.% Avicel), die Rütteldichte betrug 0,43 ± 2 mg und die Bruchfestigkeit war höher als 20 kp. Mit Hilfe des Zerfallstestes wurden die Zerfallszeiten bei verschiedenen pH-Werten ermittelt, die in Tabelle 6 aufgeführt sind.

## Tabelle 6

| Avicel PH Gew.% | pH 1 Zerfallszeit | pH 4 (Beginn des | pH 7,4 Zerfalls) |
|---|---|---|---|
| 40 | 113 min | 208 min | 280 min |
| | (9 min) | (14 min) | (11 min) |
| 50 | 100 min | 30 min | 130 min |
| | (8 min) | (9 min) | (9 min) |

Beispiel 13

Herstellung von Tabletten, bestehend aus Poly-HB, Ac-Di-Sol, Midodrin.HCl und Lactose

25 Gew.T Poly-HB, 2,5 Gew.T Ac-Di-Sol, 5 Gew.T Midodrin.HCl und 32,5 Gew.T Lactose wurden homogen vermischt und bei einem Preßdruck von 0,5 Tonnen/Tablette zu Tabletten eines Gewichtes von 65 mg, eines Durchmessers von 6 mm, einer Höhe von 1,8 mm und einer Bruchfestigkeit von 6 kp verpreßt. Die Zerfallszeiten wurden bei einem pH-Wert von 1 mittels des Zerfallstestes ermittelt. Die Tabletten zerfielen völlig innerhalb einer Minute.

**Patentansprüche**
**Patentansprüche für folgende Vertragsstaaten : AT, BE, CH DE, DK, FR, GB, IT, LI, LU, NL, SE**

1.  Preßling für die orale Applikation mit nicht retardierter Wirkstofffreisetzung, dadurch gekennzeichnet, daß er ein Homo- und/oder Copolymer der D(-)-3-Hydroxybuttersäure, mindestens ein festes Zerfallsmittel und mindestens einen festen, pharmazeutischen Wirkstoff enthält.

8

**2.** Preßling nach Anspruch 1, dadurch gekennzeichnet, daß das feste Zerfallmittel die Kapillarität des Preßlings erhöht, Feuchtigkeit absorbiert und quillt.

**3.** Preßling nach Anspruch 1 oder 2, dadurch gekennzeichnet, daß das feste Zerfallmittel mikrokristalline Cellulose, quervernetzte Natriumcarboxymethylcellulose, quervernetztes Polyvinylpyrrolidon, Natriumcarboxymethylstärke oder das Calziumsalz des Polycarboxymethylethers von Cellulose ist.

**4.** Preßling nach einem der Ansprüche 1 bis 3, dadurch gekennzeichnet, daß er in der Galenik übliche Hilfsstoffe wie Füllmittel, Bindemittel, Gleit- und Schmiermittel oder Farbstoffe enthält.

**5.** Preßling nach einem der Ansprüche 1 bis 4, dadurch gekennzeichnet, daß er Lactose enthält.

**6.** Verfahren zur Herstellung eines Preßlings mit nicht retardierter Wirkstofffreisetzung, dadurch gekennzeichnet, daß ein Homo- und/oder Copolymer der D(-)-3-Hydroxybuttersäure mit mindestens einem festen Zerfallsmittel, mindestens einem festen, pharmazeutischen Wirkstoff mit oder ohne in der Galenik übliche Hilfsstoffe durchmischt und die Mischung mit oder ohne Vorgranulierung verpreßt wird.

**7.** Verwendung von Homo- und/oder Copolymeren der D(-)-3-Hydroxybuttersäure zur Herstellung eines Preßlings gemäß Anspruch 1.

**Patentansprüche für folgende Vertragsstaaten : ES, GR**

**1.** Verfahren zur Herstellung eines Preßlings mit nicht retardierter Wirkstofffreisetzung, dadurch gekennzeichnet, daß ein Homo- und/oder Copolymer der D(-)-3-Hydroxybuttersäure mit mindestens einem festen Zerfallsmittel, mindestens einem festen, pharmazeutischen Wirkstoff mit oder ohne in der Galenik übliche Hilfsstoffe durchmischt und die Mischung mit oder ohne Vorgranulierung verpreßt wird.

**2.** Verfahren nach Anspruch 1, dadurch gekennzeichnet, daß als festes Zerfallsmittel ein Zerfallsmittel, das die Kapillarität des Preßlings erhöht, Feuchtigkeit absorbiert und quillt, eingesetzt wird.

**3.** Verfahren nach Anspruch 1 oder 2, dadurch gekennzeichnet, daß als festes Zerfallsmittel mikrokristalline Cellulose, quervernetzte Natriumcarboxymethylcellulose, quervernetztes Polyvinylpyrrolidon, Natriumcarboxymethylstärke oder das Calziumsalz des Polycarboxymethylethers von Cellulose eingesetzt wird.

**4.** Verfahren nach einem der Ansprüche 1 bis 3, dadurch gekennzeichnet, daß außer dem Homo- oder Copolymer der D(-)-3-Hydroxybuttersäure, dem Zerfallsmittel und dem oder den festen pharmazeutischen Wirkstoffen in der Galenik übliche Hilfsstoffe wie Füllmittel, Bindemittel, Gleit- und Schmiermittel oder Farbstoffe eingesetzt werden.

**5.** Verfahren nach einem der Ansprüche 1 bis 4, dadurch gekennzeichnet, daß außer dem Homo- oder Copolymer der D(-)-3-Hydroxybuttersäure dem Zerfallsmittel und dem oder den festen pharmazeutischen Wirkstoffen Lactose eingesetzt wird.

**6.** Verwendung von Homo- und/oder Copolymeren der D(-)-3-Hydroxybuttersäure zur Herstellung eines Preßlings gemäß Anspruch 1.

**Claims**
**Claims for the following Contracting States: AT, BE, CH, DE, DK, FR, GB, IT, LI, LU, NL, SE**

**1.** A tablet for oral application with unretarded release of the active ingredient characterized in that it contains a homo- and/or copolymer of D(-)-3-hydroxybutyric acid, at least one solid disintegration agent and at least one solid pharmaceutical active ingredient.

**2.** A tablet as claimed in Claim 1 characterized in that the solid disintegration agent raises the tablet's capillarity, absorbs moisture and swells.

9

3. A tablet as claimed in Claim 1 or 2 characterized in that the solid disintegration agent is microcrystalline cellulose, cross-linked sodium carboxymethyl cellulose, cross-linked polyvinylpyrrolidone, sodium carboxymethyl starch, or calcium salt of polycarboxymethylether of cellulose.

4. A tablet as claimed in any one of claims 1 to 3 characterized in that it contains adjuvants common in galenics such as fillers, excipients, lubricants, intestinal lubricants or colorants.

5. A tablet as claimed in any one of claims 1 to 4 characterized in that it contains lactose.

6. A method for producing a tablet which does not retard the release of the active ingredient characterized in that a homo- and/or copolymer of D(-)-3-hydroxybutyric acid is intermixed with at least one solid disintegration agent, at least one solid pharmaceutical active ingredient with or without adjuvants which are common in galenics, and that the mixture is pressed with or without being pregranulated.

7. An application of the homo- and/or copolymers of D(-)-3-hydroxybutyric acid for the production of a tablet in accordance with Claim 1.

**Claims for the following Contracting States : ES, GR**

1. A method for the production of a tablet with unretarded release of the active ingredient characterized in that a homo- and/or copolymer of D(-)-3-hydroxybutyric acid is intermixed with at least one solid disintegration agent, at least one solid pharmaceutical active ingredient with or without adjuvants which are common in galenics, and that the mixture is pressed with or without being pregranulated.

2. A method as claimed in Claim 1 characterized in that a disintegration agent which raises the tablet's capillarity, absorbs moisture and swells is employed as the solid disintegration agent.

3. A method as claimed in Claim 1 or 2 characterized in that microcrystalline cellulose, cross-linked sodium carboxymethyl cellulose, cross-linked polyvinylpyrrolidone, sodium carboxymethyl starch, or calcium salt of polycarboxymethylether of cellulose is employed as a solid disintegration agent.

4. A method as claimed in any one of claims 1 to 3 characterized in that adjuvants common in galenics such as fillers, excipients, lubricants, intestinal lubricants or colorants are employed in addition to the homo- and/or copolymer of D(-)-3-hydroxybutyric acid, the disintegration agent and the solid active ingredient(s).

5. A method as claimed in any one of claims 1 to 4 characterized in that lactose is employed in addition to the homo- and/or copolymer of D(-)-3-hydroxybutyric acid, the disintegration agent and the solid active ingredient(s).

6. An application of homo- and/or copolymers of D(-)-3-hydroxybutyric acid for the production of a tablet in accordance with Claim 1.

**Revendications**

**Revendications pour les Etats contractants suivants : AT, BE, CH, DE, DK, FR, GB, IT, LI, LU, NL, SE**

1. Comprimé destiné à l'administration orale, avec libération non-retardée du principe actif, caractérisé par le fait qu'il contient un homopolymère et/ou un copolymère de l'acide butyrique D(-)-3-hydroxy, au moins un produit de décomposition solide et au moins un principe d'activité pharmaceutique de forme solide.

2. Comprimé selon revendication 1, caractérisé par le fait que le produit de décomposition solide accroît la capillarité du comprimé, absorbe l'humidité et gonfle.

3. Comprimé selon revendication 1 ou 2, caractérisé par le fait que le produit de décomposition solide est constitué par de la cellulose microcristalline, de la cellulose carboxyméthylique de sodium à réticulation transversale, de la polypyrrolidone de vinyle à réticulation transversale, de l'amidon carboxyméthylique de sodium ou encore le sel calcique du polyéther carboxyméthylique de la cellulose.

4. Comprimé selon l'une des revendications 1 à 3, caractérisé par le fait qu'il contient des substances auxiliaires habituellement mises en oeuvre en galénique, telles que des matières de remplissage, des liants, des lubrifiants ou des colorants.

5. Comprimé selon l'une des revendications 1 à 4, caractérisé par le fait qu'il contient du lactose.

6. Procédé de fabrication d'un comprimé à libération non-retardée du principe actif, caractérisé par le fait qu'il contient un homopolymère et/ou un copolymère de l'acide butyrique D(-)-3-hydroxy avec au moins un produit de décomposition solide et au moins un principe d'activité pharmaceutique de forme solide, mélangé ou non à des substances auxiliaires habituellement mises en oeuvre en galénique, et que ce mélange est comprimé avec ou sans prégranulation.

7. Utilisation d'homopolymères et/ou de copolymères de l'acide butyrique D(-)-3-hydroxy dans la production d'un comprimé selon revendication 1.

**Revendications pour les Etats contractants suivants : ES, GR**

1. Procédé de fabrication d'un comprimé à libération non-retardée du principe actif, caractérisé par le fait qu'il contient un homopolymère et/ou un copolymère de l'acide butyrique D(-)-3-hydroxy avec au moins un produit de décomposition solide et au moins un principe d'activité pharmaceutique de forme solide, mélangé ou non à des substances auxiliaires habituellement mises en oeuvre en galénique, et que ce mélange est comprimé avec ou sans prégranulation.

2. Procédé selon revendication 1, caractérisé par le fait que l'on utilise, comme produit de désagregation solide, un agent capable d'accroître la capillarité du comprimé, d'absorber l'humidité et de gonfler.

3. Procédé selon revendication 1 ou 2, caractérisé par le fait que l'on utilise comme produit de décomposition solide de la cellulose microcristalline, de la cellulose carboxyméthylique de sodium à réticulation transversale, de la polypyrrolidone de vinyle à réticulation transversale, de l'amidon carboxyméthylique de sodium ou encore le sel calcique du polyéther carboxyméthylique de la cellulose.

4. Procédé selon l'une des revendications 1 à 3, caractérisé par le fait qu'il contient, outre l'homopolymère ou le copolymère de l'acide butyrique D(-)-3-hydroxy, le produit de décomposition et le ou les principes pharmaceutiquement actifs solides, des substances auxiliaires habituellement mises en oeuvre en galénique, telles que des matières de remplissage, des liants, des lubrifiants ou des colorants.

5. Comprimé selon l'une des revendications 1 à 4, caractérisé par le fait qu'il contient, outre l'homopolymère ou le copolymère de l'acide butyrique D(-)-3-hydroxy, le produit de décomposition et le ou les principes pharmaceutiquement actifs solides, du lactose.

6. Utilisation d'homopolymères et/ou de copolymères de l'acide butyrique D(-)-3-hydroxy dans la production d'un comprimé selon revendication 1.